(19) Europäisches Patentamt
European Patent Office
Office européen des brevets

(11) **EP 1 821 391 A1**

(12) **EUROPEAN PATENT APPLICATION**

(43) Date of publication:
**22.08.2007 Bulletin 2007/34**

(21) Application number: **07290204.2**

(22) Date of filing: **16.02.2007**

(51) Int Cl.:
*H02K 7/00* (2006.01)  *H02P 6/08* (2006.01)
*H02P 6/14* (2006.01)  *H02P 23/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **18.02.2006 KR 20060015915**

(71) Applicants:
• **Lee, I-Soo**
**1475, Yeonje-gu**
**Busan**
**611-803 (KR)**

• **Sim, Young-Suk**
**1475, Yeonje-gu**
**Busan**
**611-803 (KR)**

(72) Inventor: **Lee, I-Soo**
**1475, Yeonje-gu, Busan (KR)**

(74) Representative: **Bloch, Gérard et al**
**Cabinet Bloch & Associés**
**23bis, rue de Turin**
**75008 Paris (FR)**

(54) **Over-unity energy motor-generator**

(57)      An over-unity motor-generator is provided. The over-unity motor-generator includes a motor circuit unit, a motor-generator unit for generating an electric energy by rotating by an electric energy, and a generator circuit unit for commutating an alternating current generated from the motor-generator unit and outputting a direct current. The motor-generator unit includes a stator including a motor winding having n-phases, the motor winding being magnetized by receiving an electric power from the motor circuit unit and wound in an independent, multi-phase parallel distribution manner of an n-phase and a generator winding having 2n-phases and supplying the electric energy to the generator circuit unit, the generator winding being wound in an independent, multi-phase parallel distribution manner, a rotor having stacked silicon plates, flat permanent magnets buried in the stacked silicon plate and arranged in a radial direction, and a shaft located on a center of the stacked silicon plates, a commutation encoder having detection regions and non-detection regions and disposed on an end of the shaft of the rotor, and 2n-number photo sensors for transmitting an optical sensor signal to the motor circuit unit by, when the commutation encoder rotates together with the shaft, being turned on at the detection regions and off at the non-detection regions.

Fig. 2

**Description**

## BACKGROUND OF THE INVENTION

### Field of the Invention

The present invention relates to a motor-generator that can covert an electric energy into a rotational motion and further convert an energy generated by the rotational motion into an electric energy, and more particularly, to a motor-generator using an over-unity brushless direct current (BLDC) motor.

### Description of the Related Art

Likewise a direct current motor having a brush, since a BLDC motor also does not have a mechanical contact point, no noise is generated and thus the service life thereof increases. Therefore, the BLDC motor is widely used in a variety of fields such as industrial machines, home appliance, transportation, and the like. The BLDC motor uses a hole sensor as a position sensor of a rotor to control a phase of an electric current applied to a stator winding in response to a polarity of the rotor formed of permanent magnets.

Recently, considering the exhaust of petroleum resources and environmental pollution problem, endeavors for finding out a clean energy have been done. Especially, in an automobile field causing the major environment pollution problem, an electric vehicle has been developed.

Therefore, when the over-unity BLDC motor is designed to be used as not only a motor but also a generator of the electric vehicle, the efficient use of the energy will be possible.

## SUMMARY OF THE INVENTION

Accordingly, the present invention is directed to an over-unity energy motor-generator that can fulfill the above-described need.

An object of the present invention is to provide an over-unity energy motor-generator that can, when an electric energy is supplied thereto, provide a rotational force by running as a BLDC motor and generate an electric energy using the rotational force.

Additional advantages, objects, and features of the invention will be set forth in part in the description which follows and in part will become apparent to those having ordinary skill in the art upon examination of the following or may be learned from practice of the invention. The objectives and other advantages of the invention may be realized and attained by the structure particularly pointed out in the written description and claims hereof as well as the appended drawings.

To achieve these objects and other advantages and in accordance with the purpose of the invention, as embodied and broadly described herein, there is provided an over-unity motor-generator including: a motor circuit unit; a motor-generator unit for generating an electric energy by rotating by an electric energy; and a generator circuit unit for commutating an alternating current generated from the motor-generator unit and outputting a direct current, wherein the motor-generator unit includes: a stator including a motor winding having n-phases, the motor winding being magnetized by receiving an electric power from the motor circuit unit and wound in an independent, multi-phase parallel distribution manner of an n-phase and a generator winding having 2n-phases and supplying the electric energy to the generator circuit unit, the generator winding being wound in an independent, multi-phase parallel distribution manner; a rotor having stacked silicon plates, flat permanent magnets buried in the stacked silicon plate and arranged in a radial direction, and a shaft located on a center of the stacked silicon plates; a commutation encoder having detection regions and non-detection regions and disposed on an end of the shaft of the rotor; and 2n-number photo sensors for transmitting an optical sensor signal to the motor circuit unit by, when the commutation encoder rotates together with the shaft, being turned on at the detection regions and off at the non-detection regions.

The number of the detection regions of the commutation encoder is determined by the following equation 1, an angle of the width of the detection regions are determined by the following equation 2, an arrangement angle of the photo sensors are determined by the following equation 3, and an arrangement angle of the n-phase photo sensor is determined by the following equation 4:

## [Equation 1]

$$DN = PN \div 2$$

where, DN is the number of the detection regions and PN is the number of polarities of the rotor,
[0012]

## [Equation 2]

$$\Theta = \pi \div PN \div (MP+GP) \times (MP+GP-1)$$

where, PN is the number of polarities of the rotor, the number of phases of the motor, and GP is the number of phases of the generator,
[0014]

## [Equation 3]

$$\omega = \pi \div PN$$

where, PN is the number of polarities of the rotor, and
## [Equation 4]

$$\omega_n = \pi \div PN \div GP$$

where, PN is the number of polarities of the rotor and GP is the number of phases of the generator.
The stator may further include: flux dividing slots each having a relatively narrow width, the flux dividing slots being formed between motor winding slots and generator winding slots; and cancel eliminating slot each having a relatively narrow width, the cancel eliminating slots being formed between the adjacent generator winding slots.
The motor generator unit may further include a velocity encoder for detecting rotational velocity of the rotor.
The motor circuit unit may include: a direct current power supply unit using a direct current power by commutating an alternating current power or is formed of a battery to supply the direct current power; a power switching unit having H-bridges corresponding to the phases, four power semiconductor devices for each phase being connected to the motor winding, two of the semiconductor devices being alternately turned on and off according to a control signal to supply the direct current power to the motor winding; a polarity control unit receiving an optical sensor signal generated by the commutation encoder from the photo sensor and supplying the control signal for establishing the electronic rectifier to the power switching unit; and a PWM (pulse width modulation) control unit for generating a PWM signal for controlling the rotational velocity according to a control signal of a velocity control unit and a command value of a control input unit and transmitting the PWM signal to the power switching unit.
It is to be understood that both the foregoing general description and the following detailed description of the present invention are exemplary and explanatory and are intended to provide further explanation of the invention as claimed.

## BRIEF DESCRIPTION OF THE DRAWINGS

The accompanying drawings, which are included to provide a further understanding of the invention and are incorporated in and constitute a part of this application, illustrate embodiment(s) of the invention and together with the description serve to explain the principle of the invention. In the drawings:
FIG. 1 is a block diagram of an over-unity energy motor-generator according to an embodiment of the present invention;

FIG. 2 is a schematic view of a stator of the over-unity energy motor-generator of FIG. 1;

FIG. 3 is a view of a stator winding of the over-unity energy motor-generator of FIG. 1;

FIG. 4 is a schematic view of a rotor of the over-unity energy motor-generator of FIG. 1;

FIG. 5 is a schematic view of encoders used in the over-unity energy motor-generator of FIG. 1;

FIG. 6 is a view illustrating a control operation of an over-unity energy motor-generator of FIG. 1; and

FIG. 7 is a view of a signal wave of the over-unity energy motor-generator of FIG. 1.

**DETAILED DESCRIPTION OF THE INVENTION**

Reference will now be made in detail to the preferred embodiments of the present invention, examples of which are illustrated in the accompanying drawings. Wherever possible, the same reference numbers will be used throughout the drawings to refer to the same or like parts.

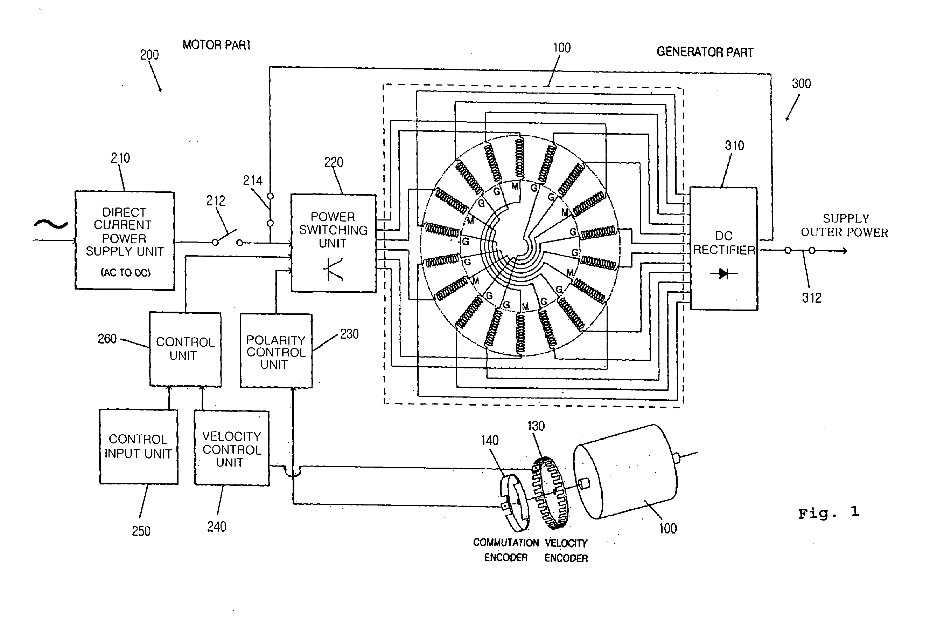

FIG. 1 is a block diagram of an over-unity energy motor-generator according to an embodiment of the present invention.

Referring to FIG. 1, an over-unity energy motor-generator of this embodiment includes a motor-generator unit 100, a motor circuit unit 200 rotating a rotor of the motor-generator unit 100 such that the motor-generator unit 100 can generate an electric energy, and a generator circuit unit 300 outputting a direct electric current by commutating an alternating electric current generated from the motor-generator unit 100.

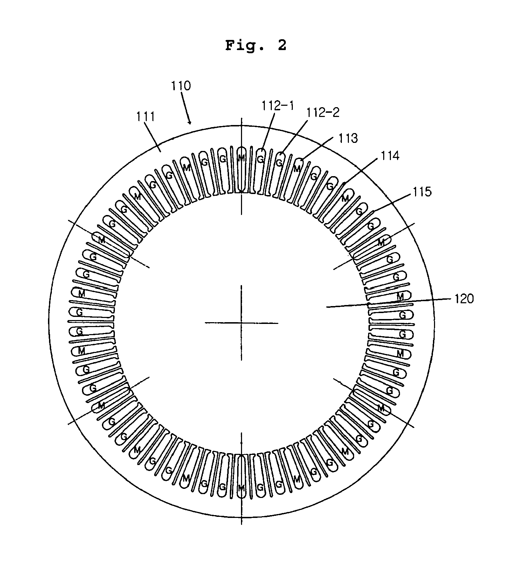

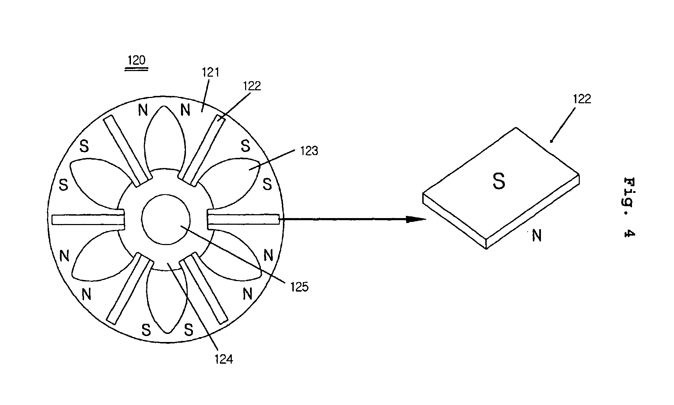

The motor-generator unit 100 includes a main body having a stator 110 (see FIG. 2) and a rotor 120 (see FIG. 4) that are located in a housing 102, and velocity and commutation encoders 130 and 140 that rotate together with a shaft 125 of the main body.

The motor circuit unit 200 includes a direct current power supply unit 210, a power switching unit 220, a polarity control unit 230, a velocity control unit 240, a control input unit 250, and a pulse width modulation (PWM) control unit 260.

The direct current power supply unit 210 supplies a direct current power to the power switching unit 220 via a switch 212 by commutating a commercial alternating current power or is formed of a battery to supply the direct current power (V+, V-) to the power switching unit 220 via the switch 212.

The power switching unit 220 turns a power semiconductor device on and off in response to a control signal to transmit an electric power of the direct current power supply unit 210 to a motor winding M of the stator 110. Here, since the power switching unit 220 is provided to supply the direct current power to the motor winding M of the stator 110, the structure thereof may vary according to the type of the motor (the number of phases of the stator winding). In order to driving one phase, four switching elements Q1-Q4 are necessary. Since these four switching element Q1-Q4 are connected to each other in the form of an H-shape, they are called an H-bridge. Therefore, the power switching unit 220 includes a plurality of H-bridges. Transistors, IGBTs, MOSFETs, and FETs are may be used as the switching elements.

The polarity control unit 230 receives an optical sensor signal from the commutation encoder 140 of the motor generator unit 100 and transmits a control signal for electrically realizing the rectifier to the power switching unit 220 to electrically realize the rectifier. The velocity control unit 112 receives an encoder signal from the velocity encoder of the motor generator and transmits a velocity control signal to the PWM control unit 260.

The control input unit 250 transmits a command signal on the rotational velocity in accordance with the manipulation of a worker. The PWM control unit 260 transmits a PWM signal for controlling the rotational velocity of the motor-generator unit 100 according to the control signal to the power switching unit 220.

The generator circuit unit 300 includes a direct current rectifier 310 commutating the alternating current generated from a generating winding G and outputting the direct current power to the power switching unit 220.

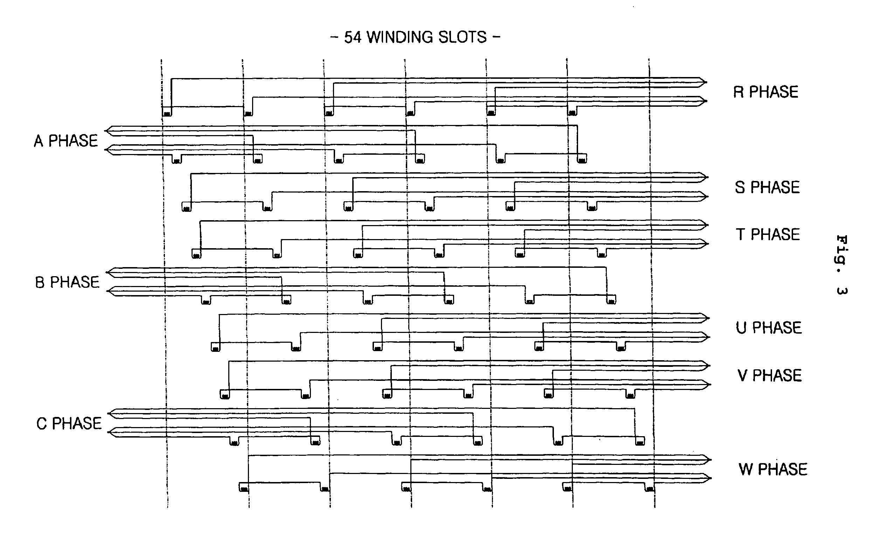

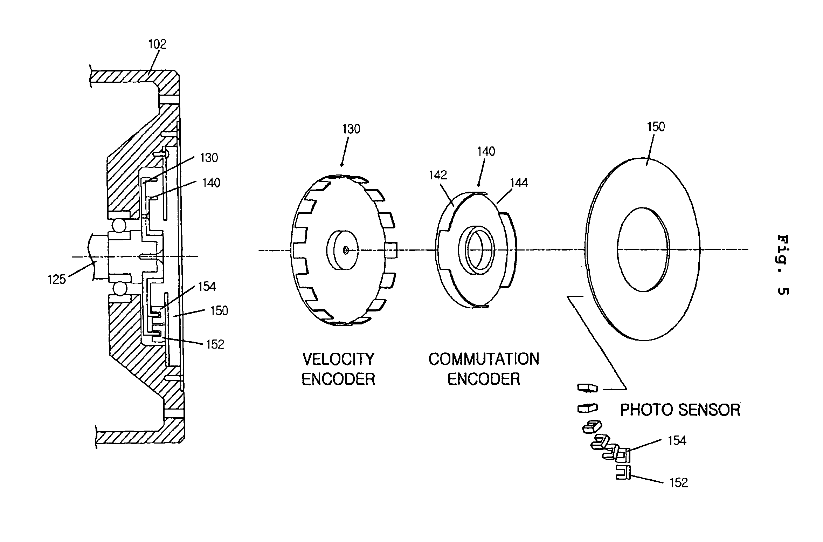

FIG. 2 is a schematic view of a stator of the over-unity energy motor-generator of FIG. 1, FIG. 3 is a view of a stator winding of the over-unity energy motor-generator of FIG. 1, FIG. 4 is a schematic view of a rotor of the over-unity energy motor-generator of FIG. 1, and FIG. 5 is a schematic view of encoders used in the over-unity energy motor-generator of FIG. 1.

The motor-generator unit 100 includes the stator 110 placed in the housing 102, the velocity encoder 130 installed at an external side of the housing, and the commutation encoder 140.

As shown in FIG. 2, the stator 110 includes a plurality of ring-shaped silicon plates 111 stacked one another, a plurality of winding slots 112-1 and 112-2, a plurality of motor winding slots 113, a plurality of flux divided slots 114, a plurality of cancel elimination slots 115, a plurality of generator winding G wound around the respective generator winding slots 112-1 and 112-2, and a plurality of motor windings M wound around the respective motor winding slots 113.

The motor windings M functions as a motor rotating the rotor 120 (i.e., motor) by receiving electric power from the motor circuit unit 200. The generator windings G functions as a generator generating electric power using the electric current induced by the rotation of the rotor 120.

In this embodiment, the total number of the winding slot and the windings is 54 that are grouped in 6 regions. 9 windings M, G, G, M, G, G, M and G (i.e., 3 motor windings M and 6 generator windings G) are arranged in each region.

Referring again to FIG. 2, the stator 110 of this embodiment is designed in an independent, multi-phase, and parallel distribution winding structure. At this point, the number of phases of the motor windings M is 2, 3, 4 ... n. The number of phases of the generator windings G is 4, 6, 8 ... 2n. The motor windings M are connected to the respective H-bridges of the power switching units 220. The generator windings G are connected to the respective direct current rectifiers 310.

When the windings of the respective phases are wound in parallel, as shown in FIG. 3, the windings are distributed and wound by the phase or polarity and connected to respective lead wires without any interconnection.

As described above, by forming the stator 110 in the independent, multi-phase parallel distribution structure, a large output can be realized at a low voltage. Since the ratio of the number of the number of the motor windings M to the number of the generator windings G is 1:2, the over-unity efficiency more than 200% can be achieved.

In addition, since the flux divided slots 114 each having a relatively narrow width are disposed between the motor winding slots 113 and the generator slots G 112-1 or 112-2, the magnet flux is divided to block a path through which the flux of the motor windings M can flow to the generator windings G so that the flux of the motor windings M can flow only to the magnetic field of the stator 110, thereby allowing the motor to effectively drive. In addition, the flux divide slot 114 maintains constantly the exciting width around the motor winding slot 113 so that the motor winding slots 113 can operate without affecting or being affected by the adjacent winding slots during driving or alternation.

The cancel elimination slots 115 each having a relatively narrow width are disposed between the generator windings 112-1 and the adjacent generator slots 112-1 to eliminate the flux cancel, thereby improving the generation efficiency.

Referring to FIG. 4, the rotor 120 of the motor-generator unit includes a plurality of silicon plates 121 stacked one another and a plurality of flat permanent magnets 122 buried in the stacked silicon plates 121 in a radial direction. At this point, the permanent magnets 122 are designed having a strong magnetic force such that a relative wide magnetic field surface can be formed and thus the magnetic flux can be concentrated on the magnetic filed surface, thereby increasing the magnetic flux density of the magnetic field surface. The number of polarities of the rotor 120 is set in response to the number of the polarities of the stator 110.

Describing the rotor 120 in more detail, six permanent magnets 122 are equally spaced apart from each other and buried in the stacked circular silicon plates 121. A non-magnet core 124 is placed on a center of the stacked circular silicon plates 121 to support the permanent magnets 122 and the silicon plates 121 and a shaft 125 is disposed through the center of the nonmagnetic core 124. The permanent magnets 122 are formed in a flat-shape and empty spaces are formed between the permanent magnets 122.

The motor using the permanent magnets is designed having a rotational force formed by the combination of the passive energy of the rotor 120 and the active energy of the stator 110. In order to the over-unity energy in the motor, it is important to enhance the passive energy of the rotor 120. Therefore, "Neodymium(Nd,Fe,B)" magnets are used in this embodiment. These magnets enlarge the magnetic field surface and allows the magnetic flux to be concentrated on the magnetic field of the rotor, thereby enhancing the magnetic flux density of the magnetic field.

Meanwhile, the commutation encoder 140 and the velocity encoder 130 are provided to control the rotation of the motor-generator unit 100. As shown in FIG. 5, the commutation encoder 140 and the velocity encoder 130 are installed on an outer depressed portion of the motor main body housing to rotate together with the rotational shaft 125 of the rotor 120.

Referring to FIG. 5, the velocity encoder 130 is a cup-shaped encoder having protrusions uniformly spaced apart from each other. The velocity encoder 130 is operated together with a photo sensor 152 installed on a sensor mount plate 150 to generate the velocity signal. The commutation encoder 140 is also a cup-shaped encoder having detecting and non-detection regions 144 and 142. The commutation encoder 140 is operated together with a photo senor installed on the sensor mount plate 150 to generator an optical sensor signal for realizing the electronic rectifier.

The number of detection regions 144 of the communication encoder 140 is determined according to the following equation 1 and an angle θ of a width of the detection region is determined according to the following equation 2.

[Equation 1]

$$DN = PNr2$$

where, DN is the number of the detection regions and PN is the number of polarities of the rotor.

[Equation 2]

$$\Theta = \pi \, WrPNr(MP+GP)s(MP+GP-1)$$

where, PN is the number of polarities of the rotor, the number of phases of the motor, and GP is the number of phases of the generator.

In addition, two photo sensors (first and second photo sensors) 154 per one phase are arranged so that they can be operated together with the commutation encoder 140. At this point, the arrangement angle ω of the first and second photo sensors 154 is determined according to the following equation 3.

[Equation 3]

$$\omega = \pi \, WrPN$$

where, PN is the number of polarities of the rotor.

Therefore, in the motor having n-phase, the arrangement angle $\omega_n$ of 2n-photosensor 154 can be determined according to the following equation 4.

[Equation 4]

$$\omega_n = \pi \, WrPNrGP$$

Where, PN is the number of polarities of the rotor and GP is the number of phases of the generator.

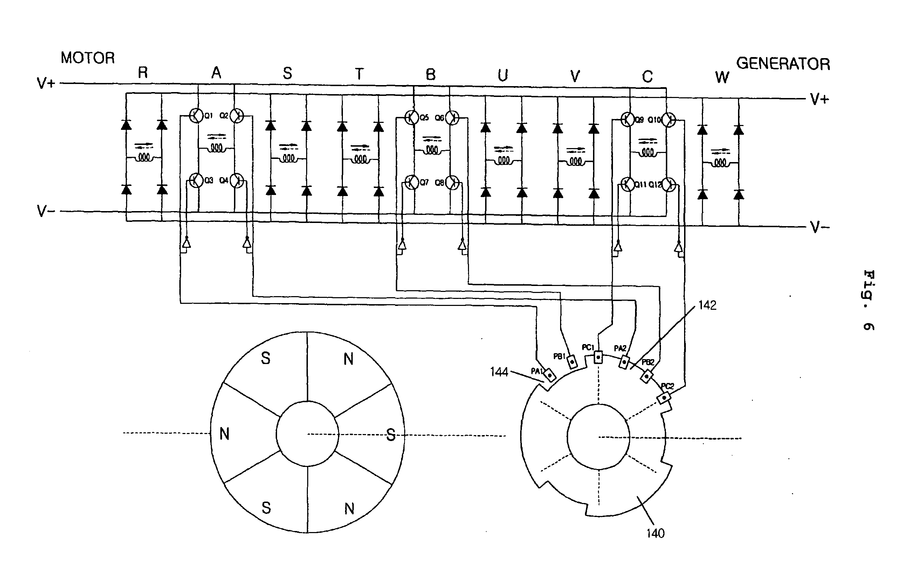

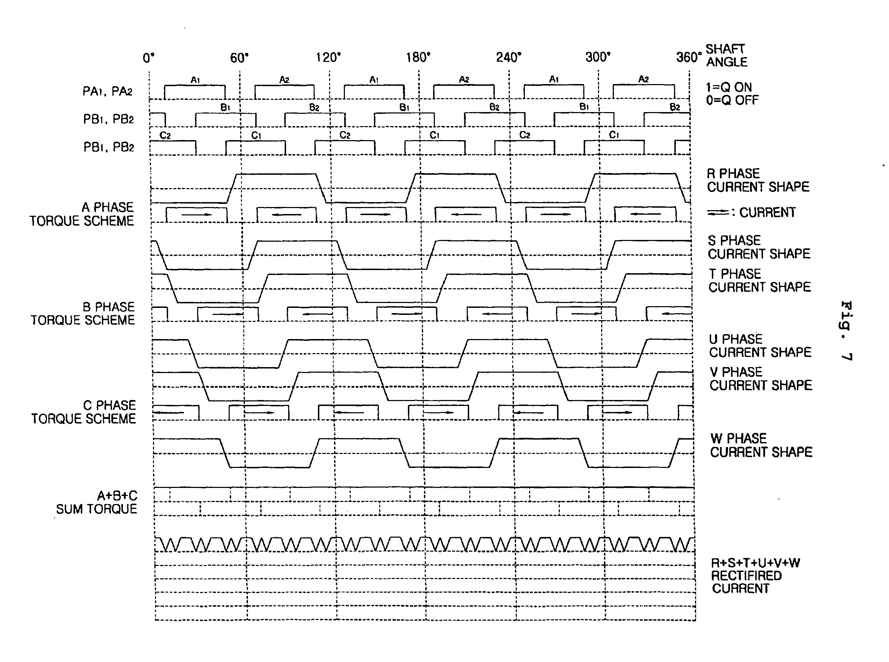

FIG. 6 is a view illustrating a control operation of an over-unity energy motor-generator of FIG. 1 and FIG. 7 is a view of a signal wave of the over-unity energy motor-generator of FIG. 1.

As shown in FIG. 6, the two photo sensors 154 of each phase is connected to switching elements of a half switch of the H-bridges of the corresponding phase to realize the electronic rectifier. At this point, the photo sensor located on the detection regions 144 of the commutation encoder turns on the half bridge of the corresponding phase of the electronic rectifier to determine the rotational direction of the rotor and the drive the motor. When the rotor rotates, an exciting width modulation is realized by the width of the detection regions 144 to turn on or off the half bridge to determine the on/off period according to which the half bridge is alternately turn on and off to effectively drive the motor-generator unit 100. In addition, when the velocity of the motor-generator unit 100 increases or decreases through the PWM control according to the control signal of the PWM control unit 260, the voltage of the motor-generator unit 260 varies so that the motor-generator unit 260 can operate at a constant velocity, thereby maintaining the predetermined require voltage.

For example, referring to FIG. 6, the direct current powers V+ and V- inputted from the motor circuit unit 200 are connected to the H-bridges of the phases A, B and C to supply electric power to the motor windings M of the respective phases. The H-bridges of the phases R, S and T and the H-bridges of the phases U, V and W commutate the corresponding electric powers generated by the generator windings G of the respective phases using the corresponding diode rectifiers and output the direct current voltage.

Describing the H-bridges of the phases A, B and C, which operate as the motor, the transistors Q1 through Q4 form the H-bridge for the phase A. The transistor Q1 and a base of the transistor Q3 are connected to the second photo sensor PA2. the transistors Q2 through Q8 form the H-bridge for the phase B. the transistor Q5 and a base of the transistor Q7 are connected to the first photo sensor PB1. The transistor Q6 and a base of the transistor Q8 are connected to the second photo sensor PB2. In addition, the transistors Q9 through Q12 form the H-bridge for the phase C. The transistor Q9 and a base of the transistor Q11 are connected to the first photo sensor PC1 and the transistor Q10 and a base of the transistor Q12 are connected to the second photo sensor PC2.

In the above-described connection state, the operation when, as shown in FIG. 6, the first photo sensor PA1 of the phase A and the first photo sensor PB1 of the phase B are located on the detection regions 144 of the commutation encoder 140 and the rest photo sensors PC1, PA2, PB2, and PC2 are located on the non-detection regions 142 will be described hereinafter.

First, since the first photo sensor PA1 of the phase A is located on the detection region 144 of the commutation encoder, the transistor Q1 is turned on while the transistor Q3 is turned off. Since the second photo sensor PA2 of the phase A is located on the non-detection region 142 of the commutation encoder, the transistor Q2 is turned off while the transistor Q4 is turned on to allow the current to flow from the transistor Q1 to the transistor Q4 via the motor winding M.

Since the first photo sensor PB1 of the phase B is located on the detection region 144 of the commutation encoder, the transistor Q5 is turned on while the transistor Q 7 is turned off. Since the second photo sensor PB2 of the phase B is located on the non-detection region 142 of the commutation encoder, the transistor Q6 is turned off while the transistor Q8 is turned on to allow the current to flow from the transistor Q5 to the transistor Q8 via the motor winding M.

If both the first and second photo sensors PA1 and PA2 are located on the non-detection regions, no electric current is applied. When the second photo sensor PA2 is located on the detection region, the transistors Q2 and Q3 are turned on to allow the current to flow from the transistor Q2 to the transistor Q3 via the motor winding M. At this point, it can be noted that the flow direction of the current flowing along the motor winding M is opposite to that when the first photo sensor PA1 is located on the detection region.

Again, when the first and second photo sensors PA1 and PA2 are located on the non-detection regions 142, no electric current is applied again. These processes are repeated to realize the alternating electric current application. At this point, the phases A, B and C undergo the alternating phase-shift-excitation to effectively drive the motor-generator unit 100. Therefore, a trapezoidal current is generated from the generator winding G such that the electric power whose voltage increases or decreases in accordance with the rotation velocity is outputted.

FIG. 7 shows torque scheme waves of the phases A, B and C throughout a rotational angle range of 0-360° of the shaft of the motor-generator, a total torque scheme of the torque scheme waves (A+B+C) throughout the rotational angle range of 0-360°, and current waves of the phases R, S and T and current waves of the phases U, V and W throughout the rotational angle range of 0-360°.

In FIG. 7, PA1 and PA2 show an on/off wave of the photo sensor of the phase A, PB1 and PB2 show an on/off wave of the photo sensor of the phase B, and PC1 and PC2 show an on/off wave of the photo sensor of the phase C.

According to the present invention, since the motor and the generator are integrated, the energy can be effectively used. Particularly, since the ratio of the motor winding to the generator winding is 1:2, the over-unity energy motor-generator can be realized.

It will be apparent to those skilled in the art that various modifications and variations can be made in the present invention. Thus, it is intended that the present invention covers the modifications and variations of this invention provided they come within the scope of the appended claims and their equivalents.

**Claims**

1. An over-unity motor-generator comprising:

   a motor circuit unit;
   a motor-generator unit for generating an electric energy by rotating by an electric energy; and
   a generator circuit unit for commutating an alternating current generated from the motor-generator unit and outputting a direct current,

   wherein the motor-generator unit comprises:

   a stator including a motor winding having n-phases, the motor winding being magnetized by receiving an electric power from the motor circuit unit and wound in an independent, multi-phase parallel distribution manner of an n-phase and a generator winding having 2n-phases and supplying the electric energy to the generator circuit unit, the generator winding being wound in an independent, multi-phase parallel distribution manner;
   a rotor having stacked silicon plates, flat permanent magnets buried in the stacked silicon plate and arranged in a radial direction, and a shaft located on a center of the stacked silicon plates;
   a commutation encoder having detection regions and non-detection regions and disposed on an end of the shaft of the rotor; and
   2n-number photo sensors for transmitting an optical sensor signal to the motor circuit unit by, when the commutation encoder rotates together with the shaft, being turned on at the detection regions and off at the non-detection regions.

2. The over-unity motor-generator of claim 1, wherein the number of the detection regions of the commutation encoder is determined by the following equation 1, an angle of the width of the detection regions are determined by the following equation 2, an arrangement angle of the photo sensors are determined by the following equation 3, and an arrangement angle of the n-phase photo sensor is determined by the following equation 4:

[Equation 1]

$$DN = PN \div 2$$

where, DN is the number of the detection regions and PN is the number of polarities of the rotor,

[Equation 2]

$$\Theta = \pi \div PN \div (MP+GP) \times (MP+GP-1)$$

where, PN is the number of polarities of the rotor, the number of phases of the motor, and GP is the number of phases of the generator,

[Equation 3]

$$\omega = \pi \div PN$$

where, PN is the number of polarities of the rotor, and

[Equation 4]

$$\omega_n = \pi \div PN \div GP$$

where, PN is the number of polarities of the rotor and GP is the number of phases of the generator.

3. The over-unity motor-generator of claim 1, wherein the stator further includes:

flux dividing slots each having a relatively narrow width, the flux dividing slots being formed between motor winding slots and generator winding slots; and
cancel eliminating slot each having a relatively narrow width, the cancel eliminating slots being formed between the adjacent generator winding slots.

4. The over-unity motor generator of claim 1, wherein the motor generator unit further comprises a velocity encoder for detecting rotational velocity of the rotor.

5. The over-unity motor generator of claim 1, wherein the motor circuit unit comprises:

a direct current power supply unit using a direct current power by commutating an alternating current power or is formed of a battery to supply the direct current power;
a power switching unit having H-bridges corresponding to the phases, four power semiconductor devices for each phase being connected to the motor winding, two of the semiconductor devices being alternately turned on and off according to a control signal to supply the direct current power to the motor winding;
a polarity control unit receiving an optical sensor signal generated by the commutation encoder from the photo sensor and supplying the control signal for establishing the electronic rectifier to the power switching unit; and
a PWM (pulse width modulation) control unit for generating a PWM signal for controlling the rotational velocity according to a control signal of a velocity control unit and a command value of a control input unit and transmitting the PWM signal to the power switching unit.

MOTOR PART 200

GENERATOR PART 100

210
DIRECT
CURRENT
POWER
SUPPLY UNIT
(AC TO DC)

212 214

220
POWER
SWITCHING
UNIT

310
DC
RECTIFIER

SUPPLY
OUTER POWER

312

300

260 CONTROL
UNIT

POLARITY
CONTROL
UNIT 230

250 CONTROL
INPUT UNIT

VELOCITY
CONTROL
UNIT

140 130 100

250 240

COMMUTATION VELOCITY
ENCODER     ENCODER

Fig. 1

EP 1 821 391 A1

Fig. 2

Fig. 3

Fig. 4

Fig. 5

Fig. 6

Fig. 7

**European Patent Office**

**EUROPEAN SEARCH REPORT**

Application Number

EP 07 29 0204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 503 485 A (NISSAN MOTOR [JP]) 2 February 2005 (2005-02-02) * paragraphs [0017], [0021], [0026]; figures 2,3 * | 1 | INV. H02K7/00 H02P6/08 H02P6/14 H02P23/00 |
| A | * figure 2 * | 2 | |
| X | * figure 1 * | 3 | |
| X | * paragraph [0021] * | 4 | |
| X | * paragraph [0008]; figure 1 * | 5 | |
| | ----- | | |
| A | EP 1 130 757 A1 (KOMATSU FUMITO [JP]) 5 September 2001 (2001-09-05) * figures 1,5,7,8 * | 3-5 | |
| | ----- | | |
| A | US 4 743 828 A (JAHNS THOMAS M [US] ET AL) 10 May 1988 (1988-05-10) * figures 2-5 * | 1,2 | |
| | ----- | | |

TECHNICAL FIELDS SEARCHED (IPC)

H02K
H02P

The present search report has been drawn up for all claims

| Place of search | Date of completion of the search | Examiner |
|---|---|---|
| Munich | 8 May 2007 | Kanelis, Konstantin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

### ANNEX TO THE EUROPEAN SEARCH REPORT
### ON EUROPEAN PATENT APPLICATION NO.

EP 07 29 0204

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2007

| Patent document cited in search report | | Publication date | Patent family member(s) | | | Publication date |
|---|---|---|---|---|---|---|
| EP 1503485 | A | 02-02-2005 | CN | 1581638 | A | 16-02-2005 |
| | | | JP | 2005057819 | A | 03-03-2005 |
| | | | US | 2005052091 | A1 | 10-03-2005 |
| EP 1130757 | A1 | 05-09-2001 | WO | 0019593 | A1 | 06-04-2000 |
| | | | TW | 221057 | B | 11-09-2004 |
| | | | US | 6424114 | B1 | 23-07-2002 |
| US 4743828 | A | 10-05-1988 | NONE | | | |

EPO FORM P0459